# EUROPEAN PATENT APPLICATION

(11) **EP 3 219 322 A1**
(43) Date of publication of application: **20.09.2017**
(21) Application number: 15859662.7
(22) Date of filing: 13.11.2015
(51) Int. Cl.: A61K 35/32, A61P 21/00, C12N 5/0775

(54) **MUSCULAR DYSTROPHY THERAPEUTIC AGENT CONTAINING PLURIPOTENT STEM CELLS DERIVED FROM DENTAL PULP**

(30) Priority: 14.11.2014 JP 2014231379
(71) Applicant: JCR Pharmaceuticals CO., LTD., Ashiya-shi, Hyogo 659-0021 (JP); Nippon Medical School Foundation, Bunkyo-ku, Tokyo 113-8602 (JP); National Center of Neurology and Psychiatry, Kodaira-shi Tokyo 187-8551 (JP)
(72) Inventor: OKADA, Takashi, Tokyo 113-8602 (JP); KASAHARA, Yuko, Kodaira-shi Tokyo 187-8551 (JP); IMAGAWA, Kiwamu, Kobe-shi Hyogo 651-2241 (JP)
(74) Representative: Isarpatent
(86) International application number: PCT/JP2015/082045
(87) International publication number: WO 2016/076434

(57) **Abstract**

Disclosed are a therapeutic agent for muscular dystrophy employing pluripotent stem cells obtained from dental pulp and a method for preparation thereof. The therapeutic agent for muscular dystrophy comprises pluripotent stem cell-enriched human dental pulp-derived cells as the active ingredient, and is prepared by a method of preparation comprising the steps of: (a) adding dental pulp-derived cells contained in a dental pulp suspension, in a culture vessel containing feeder cells whose proliferative ability is suppressed, onto a membrane having micropores that can block feeder cells from passing therethrough and supported within the vessel in a manner that avoids direct contact of the lower side face thereof with the feeder cells, and culturing the dental pulp-derived cells on the membrane while preventing direct contact with the feeder cells, and (b) recovering the cells having grown on the membrane as the pluripotent stem cell-enriched human dental pulp-derived cells.

## Description

### TECHNICAL FIELD

The present invention relates to a therapeutic agent for muscular dystrophy using pluripotent stem cells obtained from dental pulp, and more specifically, to a therapeutic agent for muscular dystrophy using pluripotent stem cells that can be prepared by culturing cells obtained from dental pulp.

### BACKGROUND ART

Muscular dystrophy is roughly classified into two groups: Duchenne muscular dystrophy (DMD) and Becker muscular dystrophy (BMD). Occurring in one out of 3,500 male newborns, DMD is one of the genetic disorders with the highest frequency. Patients of DMD at first exhibit symptoms of lowered muscular power in their infancy, and then suffer constantly progressing muscular atrophy until eventual death at the age of around 20. At present, no effective therapeutic drug for DMD is available, and therefore development of a therapeutic drug for it is craved by patients of all over the world. In BMD, the age of onset of the disease is largely in the adulthood, and although a mild loss of muscular power of the patients is observed after its onset, they can live longer than DMD patients. It has been revealed through the isolation of dystrophin gene that both DMD and BMD are caused by an abnormality occurring in the dystrophin gene, the causative gene for them (Non-patent Document 1).

As a method of treating DMD, there has been proposed a method in which the loss of muscular power of the patient is blocked, by employing an antisense oligonucleotide having a sequence complementary, and thus capable of binding, to messenger mRNA for dystrophin, and inducing with it such a splicing that leads to skipping of one or more of abnormality-containing exons of a dystrophin gene, thereby allowing expression of a dystrophin that is partially functional, though less functional than normal one (Patent Documents 1-3, Non-patent Documents 2, and 3). Such a method of treatment that uses an antisense oligonucleotide, however, has not been put to practical use.

Human mesenchymal stem cells are stem cells occurring in mesenchymal tissues and have a potential to differentiate into many types of cells such as osteocytes, cardiomyocytes, adipocytes, and so on. It is known that mesenchymal stem cells can be obtained from such tissues as bone marrow, adipocytes, placental tissue or cord tissue, dental pulp and the like (Patent Documents 4-7). Focusing on this differentiation potential of human mesenchymal stem cells, a trial has been performed to repair pathologically affected part of skeletal muscles utilizing human mesenchymal stem cells on DMD model mice (Non-patent Document 4). And based on such findings as the above, discussions have been made about the feasibility of a treatment of DMD with human mesenchymal stem cells (Non-patent Document 5). Further, feasibility is being studied of a treatment of DMD with myogenic cells generated by in vitro differentiation of human mesenchymal stem cells (Non-patent Document 6).

The dental pulp, which is a loose fibrous connective tissue that fills the pulp cavity of a tooth, is divided into the coronal pulp and the radicular pulp based on their respective location. While mesenchymal stem cells occur in dental pulp, which is a mesenchymal tissue, it is also reported that mesenchymal stem cells collected from dental pulp do not differentiate into adipocytes, suggesting existence of some stem other cells than mesenchymal stem cells (Patent Document 8). Further, it has been reported that stem cells obtained from dental pulp of deciduous teeth differ in their properties from stem cells obtained from permanent teeth, such as their high expression of FGF2, TGF-β, collagen I and collagen III as compared with those obtained from the dental pulp of permanent teeth (Patent Document 9). In addition, there also is a report which indicates that pluripotent stem cells obtained from dental pulp differ from mesenchymal stem cells obtained from bone marrow in their properties concerning their induced differentiation into osteoblasts (Patent Document 10).

As to a method of obtaining stem cells, e.g. mesenchymal stem cells, from dental pulp, there is a following report (Patent Document 11): The tissue obtained through fracturing an extracted tooth is treated with type I collagenase and dispase, and passed through a filter to remove cell aggregates and thus provide a cell suspension. The cells then are allowed to grow in a culture flask using a DMEM medium containing 20% FBS. The cells that have grown and adhered to the inner surface of the flask are detached by trypsin treatment and recovered. The cells thus recovered are considered to be mesenchymal stem cells. Further, another method for obtaining stem cells from dental pulp is also reported, in which a dental pulp suspension prepared by rupturing a tooth is cultured in a feeder cells-culture vessel containing feeder cells whose proliferative ability is suppressed, on a membrane having micropores that can block the feeder cells from passing through them and supported in the feeder cells-culture vessel in a manner to keep the lower side face of the membrane from contacting the feeder cells, thereby preventing direct contact with the feeder cells, and the cells that have proliferated on the membrane are recovered (Patent Document 12). Still further, there is a report of serum-free medium for culturing mesenchymal stem cells obtained from dental pulp (Patent Document 13).

Studies have also been conducted for feasibility of a treatment of DMD using such dental pulp-derived mesenchymal stem cells, and administration of dental pulp-derived stem cells to DMD model animals has been shown to provide stabilization of the symptoms (Non-patent Document 7).

### PRIOR ART DOCUMENTS

### PATENT DOCUMENTS

- [Patent Document 1]: JP2000-325085
- [Patent Document 2]: JP2002-010790
- [Patent Document 3]: JP2002-325582
- [Patent Document 4]: US5486359
- [Patent Document 5]: JP2004-129549
- [Patent Document 6]: JP2004-210713
- [Patent Document 7]: JP2010-252778
- [Patent Document 8]: WO 2002/007679
- [Patent Document 9]: JP2010-268715
- [Patent Document 10]: JP2004-201612
- [Patent Document 11]: JP2010-268715
- [Patent Document 12]: WO 2013/ 146992
- [Patent Document 13]: JP2011-177140

### NON-PATENT DOCUMENTS

- [Non-patent Document 1]: Koenig M. et al., Cell. 50: 509-517 (1987)
- [Non-patent Document 2]: van Deutekom JC. et al., N Engl J Med. 357: 2677-86 (2007)
- [Non-patent Document 3]: Goemans NM. et al., N Engl J Med. 364: 1513-22 (2011)
- [Non-patent Document 4]: DE Bari C. et al., J Cell Biol. 160: 909-18 (2003)
- [Non-patent Document 5]: Markert CD. et al., PM R. 1: 547-59 (2009)
- [Non-patent Document 6]: Nitahara-Kasahara Y. et al., Mol Ther. 20 : 168-77 (2012)
- [Non-patent Document 7]: Kerkis I. et al., J Transl Med. 6: 35 (2008)

### SUMMARY OF INVENTION

### TECHNICAL PROBLEM

Against the above background, the objective of the present invention is to provide an agent for treatment of muscular dystrophy employing pluripotent stem cells obtained from dental pulp.

### SOLUTION TO PROBLEM

As a result of intense studies directed to the above objective, the present inventors found that the motor function of muscular dystrophy model animals could be improved by intravenous administration of the animals with pluripotent stem cells obtained from dental pulp, and completed the present invention based thereon. Thus the present invention provides what follows.
(1) A therapeutic agent for muscular dystrophy comprising as an active ingredient pluripotent stem cell-enriched human dental pulp-derived cells.
(2) The therapeutic agent for muscular dystrophy according to 1 above that suppresses inflammation of muscles accompanying muscular dystrophy.
(3) The therapeutic agent for muscular dystrophy according to 1 or 2 above, wherein the pluripotent stem cell-enriched human dental pulp-derived cells are prepared by a method of preparation comprising the steps of:
   (a) adding dental pulp-derived cells contained in a dental pulp suspension, in a culture vessel containing feeder cells whose proliferative ability is suppressed, onto a membrane having micropores that can block feeder cells from passing therethrough and supported within the vessel in a manner that avoids direct contact of the lower side face thereof with the feeder cells, and culturing the dental pulp-derived cells, on the membrane while preventing direct contact with the feeder cells, and
   (b) recovering the cells having grown on the membrane as the pluripotent stem cell-enriched human dental pulp-derived cells.
(4) The therapeutic agent for muscular dystrophy according to 1 or 2 above, wherein the pluripotent stem cell-enriched human dental pulp-derived cells are prepared by a method of preparation comprising the steps of:
   (a) adding dental pulp-derived cells contained in a dental pulp suspension, in a first culture vessel containing feeder cells whose proliferative ability is suppressed, onto a membrane having micropores that can block feeder cells from passing therethrough and supported within the first culture vessel in a manner that avoids direct contact of the lower side face thereof with the feeder cells, and culturing the dental pulp-derived cells, on the membrane while preventing direct contact with the feeder cells,
   (b) recovering the cells having grown on the membrane,
   (c) culturing the recovered cells, in a second culture vessel containing feeder cells whose proliferative ability is suppressed, on a membrane having micropores that can block feeder cells from passing therethrough and supported within the second culture vessel in a manner that avoids direct contact of the lower side face thereof with the feeder cells while preventing direct contact with the feeder cells, and recovering the cells having grown on the membrane as the pluripotent stem cell-enriched human dental pulp-derived cells.
(5) The therapeutic agent for muscular dystrophy according to 4 above, wherein step (c) is repeated at least once.
(6) The therapeutic agent for muscular dystrophy according to one of 3 to 5 above, wherein the membrane is coated with fibronectin or collagen.
(7) The therapeutic agent for muscular dystrophy according to one of 3 to 6 above, wherein the feeder cells are mammalian cells whose proliferative ability is suppressed by mitomycin C.
(8) The therapeutic agent for muscular dystrophy according to 7 above, wherein the mammalian cells are NIH3T3 cells.
(9) The therapeutic agent for muscular dystrophy according to one of 3 to 8 above, wherein the culture is conducted in Dulbecco's modified Eagle medium that contains 10 to 25% fetal bovine serum and 3 to 5 mM L-alanyl-L-glutamine and whose glucose concentration is 5 to 7 mM.
(10) The therapeutic agent for muscular dystrophy according to one of 3 to 8 above, wherein the culture is conducted in Dulbecco's modified Eagle medium that contains 20% fetal bovine serum and 4 mM L-alanyl-L-glutamine and whose glucose concentration is 5.5 to 5.7 mM.
(11) The therapeutic agent for muscular dystrophy according to one of 3 to 10 above, wherein the method of preparation includes further steps of adding the cells recovered as pluripotent stem cell-enriched human dental pulp-derived cells to a fresh culture vessel at a density of 1×10³ to 2×10⁴ cells/cm², and culturing them until 70 to 100% of the bottom face of the culture vessel is occupied by them.
(12) The therapeutic agent for muscular dystrophy according to one of 3 to 10 above, wherein the method of preparation includes further steps of adding the cells recovered as pluripotent stem cell-enriched human dental pulp-derived cells to a fresh culture vessel at a density of 5×10³ to 1×10⁴ cells /cm², and culturing them until 90 to 100% of the bottom face of the culture vessel is occupied by them.
(13) The therapeutic agent for muscular dystrophy according to one of 1 to 12 above, wherein the pluripotent stem cell-enriched human dental pulp-derived cells are positive for the surface antigen markers CD29, CD44, CD73, CD90, CD105, and CD 166, and negative for CD34 and CD45.
(14) The therapeutic agent for muscular dystrophy according to one of 1 to 13 above, wherein the pluripotent stem cell-enriched human dental pulp-derived cells possess the ability to differentiate into chondrocyte and osteoblasts, and also the ability to suppress T cell proliferation.
(15) The therapeutic agent for muscular dystrophy according to one of 1 to 14 above, wherein the pluripotent stem cell-enriched human dental pulp-derived cells are administered at a dosage of 5×10⁵ to 2×10⁷ cells/kg body weight per single administration.
(16) The therapeutic agent for muscular dystrophy according to 15 above, wherein at least two administrations are made at an interval of 3 to 21 days.
(17) The therapeutic agent for muscular dystrophy according to 15 above, wherein at least two administration are made at an interval of 5 to 14 days.
(18) The therapeutic agent for muscular dystrophy according to 15 above, wherein at least two administrations are made at an interval of one week.

### EFFECTS OF INVENTION

According to the present invention, a therapeutic agent for muscular dystrophy, in particular Duchene muscular dystrophy, can be provided which contains dental pulp-derived pluripotent stem cells with stable traits.

### DESCRIPTION OF EMBODIMENTS

In the present specification, the term "dental pulp" refers to the loose fibrous connective tissue that fills the dental pulp cavity of a tooth, which is made of connective tissue containing blood vessels, nerves, and lymph vessels, and a layer of odontoblasts having the ability to deposit and repair dentin from inside. The dental pulp can be divided into the coronal pulp and the radicular pulp depending on their respective location, and the term "dental pulp" in the present invention includes at least either the coronal pulp or radicular pulp.

In the present specification, a tooth extracted to obtain dental pulp therefrom is used preferably within 24 hours after extraction, and more preferably within 12 hours after extraction. Dental pulp removed from an extracted tooth is minced with a tool such as a scissor, and then subjected to a treatment with a proteolytic enzyme. The proteolytic enzyme used for this is preferably a mixed solution of collagenase type II and dispase, and their concentrations are preferably 1 to 2 mg/mL and 3,000 to 7,000 units/mL, respectively, and more preferably about 1.5 mg/mL and about 5,000 units/mL, respectively. The temperature for the treatment with proteolytic enzymes is preferably 35 to 37°C, and the treatment is conducted for 1 to 3 hours. A dental pulp suspension then is prepared by disintegrating the proteolytic enzyme-treated dental pulp by pipetting. In order to remove the enzymes along with the supernatant, it is preferable to centrifuge the dental pulp suspension once and spin down the cells. The spun down material including the cells, after removal of the supernatant, is suspended again in a medium. The dental pulp suspension thus prepared contains not only the cells released from the dental pulp but also tissue debris of the dental pulp. In the present invention, the phrase "dental pulp-derived cells in a dental pulp suspension" means those cells contained in a dental pulp suspension along with dental pulp debris.

In the present specification, the phrase "pluripotent stem cell-enriched human dental pulp-derived cells" refers to a population of the cells derived from human dental pulp where the proportion (proportion in number) of pluripotent stem cells is increased compared with cells collected directly from human dental pulp, and the phrase includes isolated pluripotent stem cells finally prepared from human dental pulp through selection culture or the like.

In the present invention, the term "feeder cells" refers to other cells that are used in culturing dental pulp to promote the growth of the cells contained in the dental pulp. Feeder cells act to promote the growth of pluripotent stem cells by supplying nutrients and particular growth factors lacking in an original medium employed. Before use, feeder cells are treated so that their proliferative ability is suppressed. Examples of a method of such a treatment include treatment with an agent that inhibits cellular DNA replication or irradiation with X rays. As an agent that inhibits cellular DNA replication, mitomycin C can be preferably employed. As far as they can promote growth of the pluripotent stem cells contained in dental pulp, there is no particular limitation on what cells are to be employed as feeder cells, but preferred examples include NIH3T3 cells, BALB/3T3 cells, Swiss3T3, and mesenchymal stem cells, and dental pulp-derived pluripotent stem cells, among which NIH3T3 cells are particularly preferred. Where mesenchymal stem cells are employed as feeder cells, human bone marrow-derived mesenchymal stem cells may be employed which are obtainable following the procedure disclosed in US Patent No. 5486359 or the like, but other mesenchymal stem cells may also preferably be used such as those derived from human adipose tissue, dental pulp tissue, placental tissue, cord tissue, umbilical cord blood, or peripheral blood.

In the present invention, there is no particular limitation on the culture vessel (feeder cells-culture vessel) used for culturing feeder cells, insofar as it can be used to culture the mammalian cells, but such a vessel is preferred that enables the cells to grow while adhering to it. When NIH3T3 cells, mesenchymal stem cells, dental pulp-derived pluripotent stem cells or the like are used as feeder cells, the feeder cells added to the feeder cells-culture vessel adhere to the bottom face of the vessel. There is no particular limitation on the shape of a feeder cells-culture vessel, but a flat bottom, dish-typed vessel is preferable. Examples of such a flat bottom, dish-typed culture vessel includes a commercially available 24-well plate, 12-well plate, 6-well plate, and the like. The bottom face of a feeder cells-culture vessel is preferably coated with a cell adhesion glycoprotein such as fibronectin, collagen (collagen type I, type IV, and the like), laminin, and the like, or a peptide containing the cell adhesion active site (RGD sequence) of these cell adhesion glycoprotein.

Inside the feeder cells-culture vessel is attached a membrane that has micropores and yet can prevent the feeder cells from passing through them. The membrane is attached inside the feeder cells-culture vessel nearly horizontally relative to the bottom face of the culture vessel in such a manner that a sufficient space for culturing feeder cells is secured beneath it while its lower side face avoids contact with feeder cells. As the material of the membrane, polyethylene terephthalate and polycarbonate are preferable, and particularly preferred is polyethylene terephthalate. The micropores of the membrane have such pore sizes that can block the feeder cells (and preferably the pluripotent stem cells, too) from passing through them while allowing the dissolved components contained as solutes in the medium to pass through. The pore size of the micropores is preferably 0.1 to 1.5 µm, more preferably 0.2 to 1.2 µm, and still more preferably 0.4 to 1.0 µm. Because of their much bigger sizes than those of the pores, the feeder cells (and also the pluripotent stem cells) cannot pass through the membrane even if they include some floating cells, whereas the medium and the dissolved components secreted from the feeder cells, such as growth factors, can pass through the membrane. The membrane is preferably pre-coated with fibronectin, collagen (collagen Type I, Type IV, and the like), and laminin, and the like.

Culture is carried out following addition of the above-described dental pulp suspension onto this membrane. During the culture, the medium is added to the vessel as needed so as to ensure that the cells contained in the dental pulp suspension added on the membrane are completely covered with the medium. Thus, the culture is carried out with the dental pulp suspension placed on the upper side and the feeder cells on the lower side (but without contacting the membrane) of the interposed membrane. During the culture, components derived from the feeder cells, such as growth factors, are supplied also to the upper side of the membrane through the micropores of the membrane. In addition, components secreted from the tissue debris contained in the dental pulp suspension are also added to the medium. Thus, it is not preferable to remove the tissue debris by passing a dental pulp suspension through a mesh or the like in preparing a dental pulp suspension as disclosed, e.g., in Patent Documents 7 and 11, because tissue debris also, like feeder cells, may supply nutrients and unique growth factors lacking in the medium.

Culture medium used in culturing the dental pulp is preferably Dulbecco's modified Eagle medium containing 10 to 25% fetal bovine serum, 3 to 5 mM L-alanyl-L-glutamine, and 5 to 7 mM D-glucose, and more preferably Dulbecco's modified Eagle medium containing 20% fetal bovine serum, 4 mM L-alanyl-L-glutamine, and 5.5 to 5.7 mM D-glucose. Table 1 shows an example of a detailed composition of such a medium. Each component can be replaced with one of its equivalents (such as its salt).
[Table 1]

**Table 1. Formulation of Dulbecco's modified Eagle medium**

| Components | mM (Range) | mM (Preferred Example) |
|---|---|---|
| Glycine (aminoacetic acid) | 0.2-0.6 | 0.4 |
| L-Arginine hydrochloride | 0.3-0.5 | 0.398 |
| L-Cystine dihydrochloride | 0.15-0.25 | 0.201 |
| L-Glutamine | 3-5 | 4 |
| L-Histidine hydrochloride monohydride | 0.15-0.25 | 0.2 |
| L-Isoleucine | 0.65-0.95 | 0.802 |
| L-Leucine | 0.65-0.95 | 0.802 |
| L-Lysine hydrochloride | 0.65-0.95 | 0.798 |
| L-Methionine | 0.15-0.25 | 0.201 |
| L-Phenylalanine | 0.2-0.6 | 0.4 |
| L-Serine | 0.2-0.6 | 0.4 |
| L-Threonine | 0.65-0.95 | 0.798 |
| L-Tryptophan | 0.06-0.1 | 0.0784 |
| L-Tyrosine disodium dihydrate | 0.2-0.6 | 0.398 |
| L-Valine | 0.65-0.95 | 0.803 |
| Choline chloride | 0.02-0.04 | 0.0286 |
| D-Calcium pantothenate | 0.007-0.009 | 0.00839 |
| Folic acid | 0.008-0.01 | 0.00907 |
| Nicotinamide | 0.03-0.05 | 0.0328 |
| Pyridoxine hydrochloride | 0.015-0.025 | 0.0196 |
| Riboflavin | 0.0008-0.0012 | 0.00106 |
| Thiamine hydrochloride | 0.01-0.014 | 0.0119 |
| i-Inositol | 0.03-0.05 | 0.04 |
| Calcium chloride (anhydrous) | 1.5-2.1 | 1.8 |
| Ferric nitrate nonahydrate | 0.0002-0.0004 | 0.000248 |
| Magnesium sulfate | 0.65-0.95 | 0.814 |
| Potassium chloride | 5-6 | 5.33 |
| Sodium bicarbonate | 40-48 | 44.05 |
| Sodium chloride | 100-120 | 110.34 |
| Sodium dihydrogen phosphate monobasic monohydrate | 0.65-0.95 | 0.906 |
| D-Glucose | 5-7 | 5.56 |
| Phenol red | 0.03-0.05 | 0.0399 |
| Sodium pyruvate | 0.8-1.2 | 1 |

In the present specification, the culture that is started after addition of a dental pulp suspension onto the membrane attached inside the feeder cells-culture vessel is referred to as "primary culture (P0 culture)" of the dental pulp-derived cells. The P0 culture is preferably carried out until a colony formed by proliferation of cells on the membrane can be observed with naked eyes. During the P0 culture, it is not required to use the same initial feeder cells, but the primary culture may be carried out changing feeder cells, for example, by transferring the membrane, together with the cells and issue debris on it, to a fresh feeder cells-culture vessel. It is desirable to perform such a transfer when the feeder cells come to detach from the bottom face of the feeder cells-culture vessel during the course of culture.

When colonies are observed forming on the membrane, the membrane is washed (with a washing solution, such as a proper medium, either identical to or different from the medium used for the culture, or a buffer solution which causes no risk of adversely influencing on the cells, and so on) to remove floating cells and tissue debris, and the cells that have formed colonies are recovered by detaching them from the surface of the membrane. Recovery of the cells may be performed by a conventional method as in the case of recovery of cultured cells adhering to the container in general, namely, by adding a proteolytic enzyme, such as trypsin, detaching and dispersing the cells that have formed colonies from the membrane, and terminating the enzyme reaction by a conventional method. The cells thus recovered are added again onto a membrane and cultured in the feeder cells-culture vessel, in the same manner as the P0 culture (but without inclusion of dental pulp tissue debris). In the present specification, this culture is referred to as "pre-expansion culture" of dental pulp-derived cells. Pre-expansion culture is preferably performed at least once.

In the P0 culture and the pre-expansion culture, pluripotent stem cells multiply on the membrane most actively than other cells originating from dental pulp. Therefore, by recovering the cells proliferating on the membrane, pluripotent stem cell-enriched human dental pulp-derived cells can be obtained, which is a population of cells containing a greatly increased proportion of pluripotent stem cells compared with other cells originally occurring in human dental pulp.

Moreover, every time when the pre-expansion culture (or the below-mentioned expansion culture) is repeated, the proportion of pluripotent stem cells in recovered pluripotent stem cell-enriched human dental pulp-derived cells rapidly increases because of the greater multiplication rate of the pluripotent stem cells. Thus, cells further enriched with pluripotent stem cells can be obtained by repeating culture cycles.

In the case where pre-expansion culture is performed multiple times, each repeated culture is performed following addition of the cell suspension obtained by recovering the colonies formed on the membrane in the preceding pre-culture onto a fresh membrane in a fresh feeder cells-culture vessel. There is no particular upper limitation on the number of cycles of pre-expansion culture conducted. As it is enough that pluripotent stem cell-enriched human dental pulp-derived cells are obtained in an amount needed for the below-mentioned expansion culture, 2-5 cycles of pre-expansion culture will be sufficient in general.

After a sufficient number of pluripotent stem cell-enriched human dental pulp-derived cells are obtained, the cells can be subjected to expansion culture. Expansion culture can be repeated until a necessary number of pluripotent stem cell-enriched human dental pulp-derived cells are obtained.

In the present specification, the term "expansion culture" refers to a culture conducted in the absence of feeder cells to increase the number of pluripotent stem cell-enriched human dental pulp-derived cells (in particular, pluripotent stem cells). Pluripotent stem cells, in general, come to actively grow even in the absence of feeder cells where the cell density in a culture vessel at the start of cell culture exceeds a certain level. Such a density of cell is at least 500 cells/cm² in many cases, preferably not less than 1,000 cells/cm², more preferably not less than 3,000 cells/cm², and still more preferably not less than 5,000 cells/cm². The cell density here can be calculated by dividing the number of living cells in the cell suspension seeded in the culture vessel at the start of expansion culture by the area of the bottom face of the culture vessel.

In expansion culture, pluripotent stem cells are allowed to adhere to the culture vessel and grow there. Thus, a culture vessel used in expansion culture is that which permits culturing of mammalian cells while letting them adhere thereto, whose bottom face is preferably coated with a cell adhesion glycoprotein such as fibronectin, collagen (e.g., collagen type I, type IV), laminin and the like, or a peptide containing the cell adhesion active site of such a cell adhesion glycoprotein (RGD sequence). As such vessels, commercially available culture vessels for animal cells can be used. There is no particular limitation on the shape of a culture vessel used in expansion culture insofar as it enable culturing of cells while allowing them to adhere to it. Though vessels of a flat-bottomed dish type are preferable, those of a roller bottle type may also be used.

In expansion culture, the cells are seeded in the culture vessel so that the cell density at the start of culture is preferably 1,000 to 20,000 cells/cm², more preferably 3,000 to 15,000 cells/cm², still more preferably 5,000 to 10,000 cells/cm². And the culture is continued either until the proportion of the bottom face of the culture vessel occupied by cells reaches preferably to 70 to 100%, more preferably to 80 to 100%, and still more preferably to 90 to 100%, or preferably for 5 to 10 days, more preferably for 6 to 8 days, and still more preferably for 7days. The cells thus cultured then are recovered from the culture vessel by treatment with trypsin or the like, and expansion culture is repeated until the total number of recovered cells reaches a desired level. Pluripotent stem cells proliferate most actively also in expansion culture, and as a result, the cells obtained through expansion culture substantially consist solely of pluripotent stem cells. When they are observed in the state of adhering to the culture vessel, pluripotent stem cells obtained through expansion culture preferably exhibit a homogeneous, spindle-like shape.

In expansion culture, human dental pulp-derived pluripotent stem cells can divide not less than 40 times from the start of culture. Thus, for example, it is possible to obtain theoretically about 1×10¹² or more cells from a single pluripotent stem cell. Considering that extracted teeth, the source materials, are relatively easy to obtain, pluripotent stem cells having such a high ability to divide are promising as a supply source of pluripotent stem cells compared with other pluripotent stem cells such as bone marrow-derived mesenchymal stem cells. However, the proliferative ability of pluripotent stem cells lowers as they divide repeatedly. Therefore, when pluripotent stem cells having particularly high proliferative ability are required, the number of expansion culture cycles is preferably not more than 12, more preferably not more than 10, and most preferably not more than five (e.g., 4 or 5). Pluripotent stem cells thus obtained have ability to proliferate not less than 40 times, more preferably not less than 30 times, e.g., 30 to 35 times.

In the present invention, the term "pluripotent stem cell" refers to a cell that has both ability to proliferate and the ability to differentiate into at least two types of cells. Human dental pulp-derived pluripotent stem cells obtained according to the present invention preferably have the ability to differentiate into chondrocytes and osteoblasts. Further, human dental pulp-derived pluripotent stem cells obtained according to the present invention are generally positive for CD29, CD44, CD73, CD90, CD 105 and CD 166, and negative for CD34 and CD45.

The therapeutic agent for muscular dystrophy of the present invention is administered either by intravenous drip infusion, or local injection, with the pluripotent stem cell-enriched human dental pulp-derived cells being in the form of suspended. It can be administered not only as autogeneic administration, to the person who is the very donor of the dental pulp, but also to other, non-donors as allogeneic administration.

If allogeneic administration is expected, the pluripotent stem cell-enriched human dental pulp-derived cells of the present invention may be produced on a large scale using two or more extracted teeth obtained from two or more donors, and be frozen-stored. In this case, a manufacturer, such as a pharmaceutical company, can produce a large number of those cells, store them in the frozen state as a bulk or in the form of pharmaceutical preparations, and supply them in response to requests from medical institutions. Frozen pluripotent stem cell-enriched human dental pulp-derived cells then are thawed at medical institutions and administered to patients.

The therapeutic agent for muscular dystrophy according to the present invention is made into pharmaceutical preparations by filling containers which allow freezing of their content with a suspension of pluripotent stem cell-enriched human dental pulp-derived cells (including human dental pulp-derived pluripotent stem cells finally isolated) in a bicarbonate Ringer's solution supplemented with human serum albumin and dimethylsulfoxide. Here, the term "bicarbonate Ringer's solution" refers to a type of infusion solution (Ringer's solution) containing bicarbonate ions.

The bicarbonate Ringer's solution employed here preferably contains, as electrolytes, bicarbonate ions, sodium ions, potassium ions, calcium ions, and chloride ions; whose sodium ion content is 130 to 145 mEq, osmotic pressure of 0.9 to 1.1 relative to physiological saline, and pH 6.8 to 7.8, more preferably it contains 22 to 28 mEq/L bicarbonate ions, 120 to 150 mEq/L sodium ions, 3.6 to 4.4 mEq/L potassium ions, 2.7 to 3.3 mEq/L calcium ions, and 100 to 125 mEq/L chloride ions, with osmotic pressure of 0.9 to 1.1 relative to physiological saline, and pH 6.8 to 7.8. Such a bicarbonate Ringer's solution may further contain magnesium ions and citrate ions, wherein preferable concentration of magnesium ions is 0.9 to 1.1 mEq/L and preferable concentration of citrate ions is 4.5 to 5.5 mEq/L.

An embodiment of such a Ringer's solution is one containing 135 mEq/L sodium ions, 4 mEq/L potassium ions, 3 mEq/L calcium ions, 113 mEq/L chloride ions, 1 mEq/L magnesium ions, and 5 mEq/L citrate ions. Since a bicarbonate Ringer solution is diluted, in the process of production of pharmaceutical preparations, with human serum albumin, dimethylsulfoxide and pluripotent stem cell-enriched human dental pulp-derived cells, the concentrations of those ions in the pharmaceutical preparations correspond to their rate of dilution.

The concentration of human serum albumin in the above pharmaceutical preparations is preferably 0.1 to 10 W/V%, and more preferably 3 to 8 W/V%. Likewise, the concentration of dimethylsulfoxide in the above pharmaceutical preparations is preferably 8 to 12 W/V%.

Further, the density of pluripotent stem cell-enriched human dental pulp-derived cells in the above pharmaceutical preparations is preferably 1×10⁵ to 1×10⁸ cells/mL, and more preferably 1×10⁶ to 1×10⁷ cells/mL.

As described above, the therapeutic agent for muscular dystrophy in the form of pharmaceutical preparation, which is provided by filling containers that allow freezing of their content with a suspension of pluripotent stem cell-enriched human dental pulp-derived cells (including human dental pulp-derived pluripotent stem cells finally isolated) in bicarbonate Ringer's solution supplemented with human serum albumin and dimethylsulfoxide, is stored in liquid nitrogen or its atmosphere, and conveyed to a medical institution in response to its request while being kept in liquid nitrogen or its atmosphere. The therapeutic agent then is thawed in the medical institution and intravenously administered to a patient, directly or after added to a drip infusion solution.

While the therapeutic agent for muscular dystrophy according to the present invention may be used either as a therapeutic agent for Duchenne muscular dystrophy or for Becker muscular dystrophy, it is particularly useful as a therapeutic agent for Duchenne muscular dystrophy. The therapeutic agent for muscular dystrophy according to the present invention can also be used prophylactically for genetic carriers whose muscular dystrophy has not yet developed, for the purpose of preventing occurrence of inflammation of muscles which accompanies muscular dystrophy. Further, the therapeutic agent for muscular dystrophy according to the present invention is particularly useful in ameliorating the inflammation of muscles observed in patient of muscular dystrophy. Inflammation of muscles is thought to induce degeneration of muscle tissues in muscular dystrophy, and it thus is possible to suppress the development of muscle weakness caused by degeneration of muscle tissues in muscular dystrophy patients, by suppressing the inflammation of muscles with the therapeutic agent for muscular dystrophy of the present invention. The term "muscles" herein refers mainly to the skeletal muscles, the cardiac muscle, and the muscles of respiration. Thus, by suppressing inflammation of skeletal muscles, it is possible to maintain muscle strength of muscular dystrophy patients, thereby suppressing decline of their walking ability. Further, it is possible to maintain the cardiopulmonary function of muscular dystrophy patients by suppressing inflammation of the cardiac muscle and the muscles of respiration.

The therapeutic agent for muscular dystrophy according to the present invention is administered so that the pluripotent stem cell-enriched human dental pulp-derived cells are administered preferably at a dose of 5×10⁵ to 2×10⁷ cells, more preferably 1×10⁶ to 1×10⁷ cells, and still more preferably 4×10⁶ cells, per administration and per kg body weight of a patient. Further, the therapeutic agent for muscular dystrophy according to the present invention is administered preferably at least twice at an interval of 3 to 21 days, more preferably at least twice at an interval of 5 to 14 day, still more preferably at least twice at an interval of a week. In general, administration is continued until the patients' symptoms have ameliorated.

### EXAMPLES

While the present invention will be described in further detail below referring to examples, it is not intended that the present invention be limited to the examples.

### [Example 1]

### Preparation of pluripotent stem cell-enriched human dental pulp-derived cells

### (1) Provision of feeder cells

FBS (Invitrogen Inc.) and L-alanyl-L-glutamine were added to DMEM Low Glucose (Invitrogen Inc.) at their final concentration of 10% and 4 mM, respectively, and the medium thus prepared was designated DMEM (10% FBS) medium. Mitomycin C (SIGMA) was dissolved in water for injection to the concentration of 0.2 mg/mL, and the solution thus prepared was designated Mitomycin C solution. FBS (Invitrogen Inc.) and L-alanyl-L-glutamine were added to DMEM Low Glucose (Invitrogen Inc.) at their final concentration of 20% and 4 mM, respectively, and the medium thus prepared was designated DMEM (20% FBS).

NIH3T3 cells frozen stored in liquid nitrogen were taken out and thawed in a thermostatic bath set to 37 °C. The cells then were suspended in DMEM (10% FBS) medium added to them, and was centrifuged (1,500 rpm, 5 min). The supernatant was discarded, and the cells were suspended in DMEM (10% FBS) medium, seeded in a 75 cm² culture flask, and incubated at 37 °C under 5% CO₂ until the cell density reached 80 to 90%. The cells were washed with Dulbecco's phosphate buffered solution (D-PBS, Invitrogen Inc.), and then a medium prepared by adding 0.4 mL of Mitomycin C solution to 9.6 mL of DMEM (10% FBS) medium was added to the culture flask, and the cells were left undisturbed at 37 °C under 5% CO₂. After removal of the medium, the cells were washed with D-PBS, and following addition of 1 mL of a 0.25% trypsin-EDTA solution, left undisturbed for 5 to 10 minutes at 37 °C. After the detachment of cells was ensured, the reaction was terminated by addition of DMEM (10% FBS) medium. After the cells were suspended, the number of viable cells was counted on a hemacytometer. The cells were collected into a 15 mL centrifuge tube, spun down by centrifugation (1,500 rpm, 5 min), and suspended at the cell density of 1×10⁶ cells/mL in a serum solution containing 10% DMSO (v/v), and dispensed 2 ml each into cryopreservation tubes, and then were frozen at -80 °C. After stored at -80°C for more than 24 hours, the cells were transferred into liquid nitrogen and stored there. Frozen cells thus provided were used as feeder cells.

Before use, feeder cells were taken out of the liquid nitrogen and thawed, and after suspended in DMEM (20% FBS) medium added thereto, they were spun down by centrifugation (1,500 rpm, 10 min). The feeder cells then were suspended in DMEM (20% FBS) medium at a concentration of 4×10⁴ cells/mL, and the suspension was added 500 µL each to the bottom wells of 12-well cell culture insert companion plate (12-well Companion Plate, BD Biosciences Inc.) so as to let the cells adhere to the bottom face of the well.

### (2) Isolation of dental pulp

After 12 mg of collagenase type II (Calbiochem Inc.) was added to 4 ml of D-PBS and mixed, the mixture was filtered through a 0.22 µm filter. The solution thus obtained was designated Collagenase Solution. After 10,000 units of dispase (Godo Shusei Co., Ltd.) was added to 1 ml of D-PBS and mixed, the mixture was filtered through a 0.22 µm filter. The solution thus obtained was designated Dispase Solution.

An extracted tooth obtained on an informed consent was lightly washed with Ringer's solution and then transferred to a 10 cm dish. Saline was added to the dish to wash the extracted tooth, and 0.5% chlorhexidine solution then was added and stirred to sterilize the surface of the extracted tooth. The extracted tooth then was placed under a sterile environment and washed with sterilized saline until the 0.5% chlorhexidine was sufficiently removed. After removing the saline, the dental pulp was exposed by dividing the extracted tooth using sterilized dental pliers and forceps. After resected with surgical scissors, the dental pulp was transferred to a centrifuge tube and minced with surgical scissors. Then, following addition of Dispase Solution and Collagenase Solution, 150 µL each, to the centrifuge tube, ,the dental pulp tissue was sufficiently disintegrated by pipetting, and was left undisturbed for 1 to 2 hours under 37 °C.

Subsequently, the enzyme reaction was terminated by addition of 5 mL of DMEM (20% FBS) medium, and the material containing cells was spun down by centrifugation (1,500 rpm, 10 min). After removal of the supernatant, 5 mL of DMEM (20% FBS) medium was added to the spun down materials to suspend the materials including cells, which then were spun down by further centrifugation (1500 rpm, 10 min). After addition of 500 µL of DMEM (20% FBS) medium to the spun down materials, they were suspended well by pipetting to provide a suspension of dental pulp-derived cells containing tissue debris.

### (3) Primary culture of dental pulp-derived cells (P0 culture)

Fibronectin was added at a density of 1 µg/cm² onto the polyethylene terephthalate porous membrane with a pore size of 0.4 µm (track etched membrane) forming the bottom of a 12-well insert (BD Falcon Cell Culture Insert, BD Biosciences), and was left undisturbed at 37° C at least for 30 minutes so that the membrane is coated with fibronectin. The fibronectin employed here been prepared according to the method described in Horwitz B. et al, Preparation of fibronectin for therapeutic administration In:. Mosher DF, editor. New York Academic Press Inc 441-445 (1989).

The 12-well insert that had been coated with fibronectin was placed in a 12-well companion plate whose bottom wells contained the feeder cells as prepared above, and the dental pulp suspension (about 500 µL) was added onto this 12-well insert, and after addition of DMEM (20% FBS) medium so that all the cells were covered with it, primary culture (P0) was started at 37 °C under 5% CO₂. Changing the medium once every 3 to 4 days, the culture was continued until a colony was visually observed on the 12-well insert. During this period, if the feeder cells in the bottom wells were found markedly detached from the bottom face of the bottom wells, a fresh 12-well companion plate was prepared containing feeder cells that adhered to the bottom face of its bottom wells, to which the 12-well insert was transferred, and culture was continued.

A 12-well insert in which a colony was visually observed was transferred to another companion plate, and 1 ml of PBS was added to it to wash the cells that adhered to the 12-well insert and remove floating cells and tissue debris. Then, 500 µL of 0.25% trypsin-EDTA solution was added to the 12-well insert and left undisturbed for 5-10 minutes at 37 °C to detach the cells that had adhered to the porous membrane of the 12-well insert. Then, 300 µL of DMEM (20% FBS) medium was added to terminate the reaction and suspend the cells, and the cell suspension was collected into a centrifuge tube. Further, 300 µL each of DMEM (20% FBS) medium was added to the 12-well insert to suspend remaining cells, which then were collected into the same centrifuge tube. The collected cells were spun down by centrifugation (1,500 rpm, 5 min), and after removal of the supernatant, 1 ml of DMEM (20% FBS) medium was added to form a cell suspension.

### (4) Pre-expansion culture of dental pulp-derived cells

A 6-well companion plate with feeder cells added to its bottom wells was prepared, and a 6-well insert coated with fibronectin was placed on it. The cell suspension obtained by the primary culture (P0) was added to the 6-well insert, and pre-expansion culture was started at 37 °C in the presence of 5% CO₂. Exchanging DMEM (20% FBS) medium once every 3 to 4 days, culture was continued until a colony was visually observed on the 6-well insert. During this period, if the feeder cells in the bottom wells were found markedly detached from the bottom wells, a fresh 6-well companion plate was prepared containing feeder cells that adhered to the bottom face of its bottom wells, to which the 12-well insert was transferred, and culture was continued.

The 6-well insert on which a colony was visually observed was transferred to another companion plate, and the cells were washed with PBS, and 500 µL of 0.25% trypsin-EDTA solution was added. After left undisturbed for 5 to 10 minutes at 37 °C and detachment of the cells observed, 500 µL of DMEM (20% FBS) medium was added to terminate the reaction and suspend the cells, and the cell suspension was collected into a centrifuge tube. Further, the 6-well insert was washed with 500 µL of DMEM (20% FBS) medium, and the washings were collected into the same centrifuge tube. The collected cells were spun down by centrifugation (1,500 rpm, 5 min), and after removing the supernatant, 500µL of DMEM (20% FBS) medium was added to suspend the cells.

### (5) Expansion culture of dental pulp-derived cells

After the number of viable cells contained in the above cell suspension was measured on a hemacytometer, the cells were seeded at a density of 5×10³ to 1×10⁴ cells/cm² in a culture flask, and expansion culture was started in DMEM (10% FBS) medium. However, if expansion culture cannot be started because the number of viable cells was too small, pre-expansion culture was repeated until the number of cells needed to start the expansion culture was obtained. The cells seeded in the culture flask were observed at the start of expansion culture, and it was found that almost all the cells adhered to the bottom face of the culture flask and had a spindle-like shape, which confirmed that homogeneous cells was isolated.

After the cells were cultured in DMEM (10% FBS) medium until 90 to 100% confluency was reached, the cells were washed with PBS, and following addition of 0.25% trypsin-EDTA, left undisturbed for 5 to 10 minutes at 37 °C so as to detached them. DMEM (10% FBS) medium was added to terminate the reaction and suspend the cells, and the cells were collected into 15 ml centrifuge tubes. The cells thus collected were spun down by centrifugation (1500 rpm, 5 min), and after removing the supernatant, the cells were suspended in DMEM (10% FBS) medium. After measuring the number of viable cells on a hemacytometer, the cells were seeded at a density of 5×10³ to 1×10⁴ cells/cm² in a culture flask and cultured until 90 to 100% cell confluency was reached. Expansion culture was repeated 16 times, and the number of cell division cycles was calculated based on the number of the cells measured at the end of each subculture. Besides, even if the cell confluency did not reach 90 to 100%, the cells were collected and subjected to the next expansion culture when seven days had passed from the start of each expansion culture. The rate of proliferation of the cells gradually lowered since the fourth expansion culture, and the cell confluency thus never reached 90 to 100% even seven days after the start of each expansion culture.

### (6) Frozen Storage of dental pulp-derived cells

In the second expansion culture, part of the cells that had been cultured until the confluency reached 90 to 100% was frozen-stored by the following method: After washing with PBS, 0.25%, trypsin-EDTA was added to the cells and left undisturbed for 5 to 10 minutes at 37 °C to detach them. DMEM (10% FBS) medium was added to terminate the reaction and suspend the cells, and the cells then were collected into 15 mL centrifuge tubes. The cells thus collected were spun down by centrifugation (1500 rpm, 5 min), and the supernatant was removed. The cells were suspended in a FBS solution containing 10% (v/v) DMSO at a density of 1×10⁶ cells/mL. This cell suspension, 0.5 to 2 mL each, was dispensed into a cryotube, and then placed in a BICELL (Japan Freezer) that had been cooled at 4 °C in advance, and was frozen at -80°C. About 24 hours after freezing, the cells were transferred to vapor-phase liquid nitrogen and stored in it. These cells were used in the following experiments as pluripotent stem cell-enriched human dental pulp-derived cells.

### [Example 2]

### Provision of DMD model animals

For a DMD model animal, artificial fertilization was performed on a Beagle with spermatozoa of a Golden Retriever with muscular dystrophy to create carrier dogs that had an abnormal dystrophin gene in heterologous fashion. These carrier dogs then were allowed to naturally mate pure-line Beagles, and by repeating this process in at least five consecutive generations, DMD model animals were produced with a similar size to a Beagle and used in the following experiment as muscular dystrophy model dogs (herein after referred to as "muscular dystrophy dogs"). Besides, it is known that a single nucleotide substitution occurs in the splice-consensus sequence on the 3'-side of intron 6 of the dystrophin gene of the muscular dystrophy dogs (Sharp NJH. Genomics. 13: 115-21 (1991)). Because of this substitution, exon 7 is skipped during the splicing process of the mRNA from the dystrophin gene, and as the mRNA from the dystrophin gene is translated, a frameshift occurs in exon 8, which leads to termination of further translation. As a result, a fractional dystrophin is produced, which lacks the enzyme's intrinsic function, and the muscular dystrophy dogs thus exhibit similar symptoms to those observed in human DMD.

### [Example 3]

### Administration of pluripotent stem cell-enriched human dental pulp-derived cells to DMD model animals

Two dogs of a litter, 6-week old, were provided. As the first course of administration, one of the dogs (cells-administered dog) was intravenously injected with pluripotent stem cell-enriched human dental pulp-derived cells when it became 7, 8, 9, and 10 weeks old, at a dose of 4×10⁶ cells/kg (body weight), and the other dog (control dog) was intravenously injected with the same volume of physiological saline in the same manner. As the second course of administration, a dog (cells-administered dog) was intravenously injected with pluripotent stem cell-enriched human dental pulp-derived cells when it became 18, 19, 20, and 22 weeks old, at a dose of 4×10⁶ cells/kg (body weight). As it died at 14 weeks of age, no administration was possible to the control dog as the second course of administration.

### [Example 4]

### Evaluation of muscular inflammation

Using MRI image analysis on 3.0-Tesla MRI, Magnetom Trio (Siemens Medical Solutions, Inc.), cross-sectional images (fat suppression T2-weighted images) of the lower legs of the cells-administered and control dogs were taken immediately before starting the first administration of the cells or saline in the first course ("first administration of cells") and 7 days after the fourth administration of the cells or saline ("forth administration of cells"). Immediately before the start of the first administration of cells, signals indicating the presence of inflammation were widely observed in the cross-sectional images of the lower legs of both the cells-administered and the control dogs. Seven days after the fourth administration of cells, signals indicating inflammation in the cross-sectional images of the control dog were found to have strengthened as a whole, demonstrating the progress of muscular inflammation accompanying muscular dystrophy. On the other hand, while the signals indicating inflammation were also found to have strengthened as a whole in the cells-administered dog, they were weaker in degree than the control dog. These results suggested that administration of pluripotent stem cell-enriched human dental pulp-derived cells can suppress the progress of muscular inflammation in the DMD model animals.

### [Example 5]

### Evaluation of motor function

The cells-administered dog and the control dog were compared in their motor function by measuring the length of time required for them to run a distance of 15 m. Measurement of the motor function of the control dog at 11 weeks of age, i.e., after completion of the first course of administration, revealed a decline in motor function compared with the condition immediately before the start of the first course of administration. On the contrary, an improvement in motor function was observed in the cells-administered dog, at 11 weeks of age, i.e., after the completion of the first course of administration and at 22 weeks of age, i.e., after the completion of the second course of administration, in comparison with the condition immediately before the start of the first administration. In general, DMD model animals (dogs) as used in this experiment tend to exhibit decline in their motor function with aging as seen in the control dog. The above results suggested that administration of pluripotent stem cell-enriched human dental pulp-derived cells can suppress the decline of, or maintain, the motor function of DMD model animals.

### [Example 6]

### Evaluation of cardiac function

The cells-administered dog and a non-DMD normal dog of the same litter as the cell-administered dog were compared in their cardiac function by measuring the left ventricular ejection fraction based on ultrasonic imaging analysis (on Vivid S6, GE Healthcare) at 27 weeks of age, i.e., after the completion of the second course of administration. As a result, the left ventricular ejection fraction of the normal dog and the cells-administered dog were 70.7% and 61.5%, respectively, revealing that the left ventricular ejection fraction of the cells-administered dog was maintained at about 87% of the normal dog at 27 weeks of age. The left ventricular ejection fraction of severe DMD model animals tends to notably decline relative to normal dogs at 27 weeks of age, and in comparison with that, the above results suggest that administration of pluripotent stem cell-enriched human dental pulp-derived cells can suppress the decline in cardiac function of DMD model animals.

From the above results, pluripotent stem cell-enriched human dental pulp-derived cells are thought to suppress the degeneration of muscles by suppressing the progress of muscular inflammation which accompanies muscular dystrophy, and thus have the effect of suppressing the decline in motor and cardiac functions occurring in muscular dystrophy.

### INDUSTRIAL APPLICABILITY

The present invention can provide a novel therapeutic agent for muscular dystrophy employing pluripotent stem cells originating from dental pulp as the active principle.

## Claims

1. A therapeutic agent for muscular dystrophy comprising as an active ingredient pluripotent stem cell-enriched human dental pulp-derived cells.

2. The therapeutic agent for muscular dystrophy according to claim 1 that suppresses inflammation of muscles accompanying muscular dystrophy.

3. The therapeutic agent for muscular dystrophy according to claim 1 or 2, wherein the pluripotent stem cell-enriched human dental pulp-derived cells are prepared by a method of preparation comprising the steps of:
(a) adding dental pulp-derived cells contained in a dental pulp suspension, in a culture vessel containing feeder cells whose proliferative ability is suppressed, onto a membrane having micropores that can block feeder cells from passing therethrough and supported within the vessel in a manner that avoids direct contact of the lower side face thereof with the feeder cells, and culturing the dental pulp-derived cells, on the membrane while preventing direct contact with the feeder cells, and
(b) recovering the cells having grown on the membrane as the pluripotent stem cell-enriched human dental pulp-derived cells.

4. The therapeutic agent for muscular dystrophy according to claim 1 or 2, wherein the pluripotent stem cell-enriched human dental pulp-derived cells are prepared by a method of preparation comprising the steps of:
(a) adding dental pulp-derived cells contained in a dental pulp suspension, in a first culture vessel containing feeder cells whose proliferative ability is suppressed, onto a membrane having micropores that can block feeder cells from passing therethrough and supported within the first culture vessel in a manner that avoids direct contact of the lower side face thereof with the feeder cells, and culturing the dental pulp-derived cells, on the membrane while preventing direct contact with the feeder cells,
(b) recovering the cells having grown on the membrane,
(c) culturing the recovered cells, in a second culture vessel containing feeder cells whose proliferative ability is suppressed, on a membrane having micropores that can block feeder cells from passing therethrough and supported within the second culture vessel in a manner that avoids direct contact of the lower side face thereof with the feeder cells while preventing direct contact with the feeder cells, and recovering the cells having grown on the membrane as the pluripotent stem cell-enriched human dental pulp-derived cells.

5. The therapeutic agent for muscular dystrophy according to claim 4, wherein step (c) is repeated at least once.

6. The therapeutic agent for muscular dystrophy according to one of claims 3 to 5, wherein the membrane is coated with fibronectin or collagen.

7. The therapeutic agent for muscular dystrophy according one of claims 3 to 6, wherein the feeder cells are mammalian cells whose proliferative ability is suppressed by mitomycin C.

8. The therapeutic agent for muscular dystrophy according to claim 7, wherein the mammalian cells are NIH3T3 cells.

9. The therapeutic agent for muscular dystrophy according to one of claims 3 to 8, wherein the culture is conducted in Dulbecco's modified Eagle medium that contains 10 to 25% fetal bovine serum and 3 to 5 mM L-alanyl-L-glutamine and whose glucose concentration is 5 to 7 mM.

10. The therapeutic agent for muscular dystrophy according to one of claims 3 to 8, wherein the culture is conducted in Dulbecco's modified Eagle medium that contains 20% fetal bovine serum and 4 mM L-alanyl-L-glutamine and whose glucose concentration is 5.5 to 5.7 mM.

11. The therapeutic agent for muscular dystrophy according to one of claims 3 to 10, wherein the method of preparation includes further steps of adding the cells recovered as pluripotent stem cell-enriched human dental pulp-derived cells to a fresh culture vessel at a density of 1×10³ to 2×10⁴ cells/ cm², and culturing them until 70 to 100% of the bottom face of the culture vessel is occupied by them.

12. The therapeutic agent for muscular dystrophy according to one of claims 3 to 10, wherein the method of preparation includes further steps of adding the cells recovered as pluripotent stem cell-enriched human dental pulp-derived cells to a fresh culture vessel at a density of 5×10³ to 1×10⁴ cells /cm², and culturing them until 90 to 100% of the bottom face of the culture vessel is occupied by them.

13. The therapeutic agent for muscular dystrophy according to one of claims 1 to 12, wherein the pluripotent stem cell-enriched human dental pulp-derived cells are positive for the surface antigen markers CD29, CD44, CD73, CD90, CD 105, and CD 166, and negative for CD34 and CD45.

14. The therapeutic agent for muscular dystrophy according to one of claims 1 to 13, wherein the pluripotent stem cell-enriched human dental pulp-derived cells possess the ability to differentiate into chondrocyte and osteoblasts, and also the ability to suppress T cell proliferation.

15. The therapeutic agent for muscular dystrophy according one of claims 1 to 14, wherein the pluripotent stem cell-enriched human dental pulp-derived cells are administered at a dosage of 5×10⁵ to 2×10⁷ cells/kg body weight per single administration.

16. The therapeutic agent for muscular dystrophy according to claim 15, wherein at least two administrations are made at an interval of 3 to 21 days.

17. The therapeutic agent for muscular dystrophy according to claim 15, wherein at least two administration are made at an interval of 5 to 14 days.

18. The therapeutic agent for muscular dystrophy according to claim 15, wherein at least two administrations are made at an interval of one week.
